# FASCICULE DE BREVET EUROPEEN

(11) **EP 3 079 788 B1**
(45) Date de publication et mention de la délivrance du brevet: **05.02.2020**
(21) Numéro de dépôt: 14815609.4
(22) Date de dépôt: 09.12.2014
(51) Int. Cl.: B01D 15/18, C07C 51/47, C07C 57/03

(54) **PROCÉDÉ DE PURIFICATION CHROMATOGRAPHIQUE D'UN ACIDE GRAS**
VERFAHREN ZUR CHROMATOGRAPHISCHEN REINIGUNG EINER FETTSÄURE
METHOD FOR CHROMATOGRAPHIC PURIFICATION OF A FATTY ACID

(30) Priorité: 11.12.2013 FR 1362444
(43) Date de publication de la demande: 19.10.2016
(60) Demande divisionnaire: 18209325.2
(73) Titulaire: Novasep Process, 54340 Pompey (FR)
(72) Inventeur: VALÉRY, Eric, F-54420 Saulxures-lès-Nancy (FR); BLÉHAUT, Jean, F-69002 Lyon (FR)
(74) Mandataire: August Debouzy
(86) Numéro de dépôt international: PCT/EP2014/077067
(87) Numéro de publication internationale: WO 2015/086607

(56) Documents cités:
- WO-A1-94/25552
- WO-A1-98/51391
- WO-A1-2013/005048
- WO-A1-2014/108686
- US-A- 5 630 943

## Description

### DOMAINE DE L'INVENTION

La présente invention concerne un procédé chromatographique de production d'acides gras, et notamment d'acides gras polyinsaturés, tels que l'acide eicosapentaénoïque, ainsi qu'une installation adaptée à la mise en œuvre de ce procédé.

### ARRIERE-PLAN TECHNIQUE

Les acides gras, dont les acides gras polyinsaturés (en abrégé PUFA), sont des composés biologiques particulièrement importants car ils interviennent dans de nombreux processus biologiques tels que la construction et le maintien des membranes cellulaires, la synthèse d'hormones (par exemple les prostaglandines) qui jouent un rôle dans l'agrégation plaquettaire, les processus d'inflammation et la réponse immunologique, etc.

La plupart des PUFA peuvent être synthétisés par un organisme humain, à l'exception de deux familles de PUFA qui doivent être obligatoirement apportés par l'alimentation, appelés les acides gras essentiels.

Les deux familles d'acides gras essentiels sont :
- les oméga-6, qui sont particulièrement abondant dans les huiles de noix, de tournesol, de soja, de pépins de raisins ou de maïs et les volailles grasses (tel que le canard) ;
- les oméga-3 qui sont surtout présents dans les huiles de noix, dans les végétaux tel que le colza et le lin et dans les poissons gras (tels que le saumon, le thon, la sardine, le maquereau ou le hareng). Des procédés de production d'oméga-3 utilisant des cultures de micro-algues, des levures transgéniques ou de krill ont été récemment développés.

Les oméga-3 sont des PUFA particulièrement intéressants pour leurs vertus antioxydantes. Parmi ces oméga-3, l'EPA (acide eicosapentaénoïque, C20-5w3) et le DHA (acide docosahexaénoïque, C22-6w3) purifiés et leurs combinaisons enrichies sont les plus utilisés comme compléments alimentaires ou médicaments pour diminuer le taux de triglycérides, les risques cardiovasculaires, améliorer la cognition ou la vision, etc.

Des études cliniques récentes ont montré que le traitement de patients ayant un taux de triglycérides supérieur à 500 ml/dL par 4 grammes par jour d'ester éthylique d'EPA à 96 % sans DHA permettait de faire baisser le taux de triglycéride, sans faire augmenter le taux de LDL (« mauvais » cholestérol), alors que le traitement avec 4 grammes par jour d'un mélange d'esters éthyliques d'EPA et de DHA, à environ 50 % et 35 % respectivement, conduisait à une augmentation du taux de LDL concomitant à la diminution des triglycérides.

Jusqu'à présent, les compléments alimentaires de PUFA utilisés, notamment les oméga-3, sont essentiellement basés sur des mélanges contenant 30 à 60 % du mélange d'EPA et DHA. Dans les méthodes de séparation utilisées à ce jour, le mélange est obtenu par transestérification des triglycérides en esters éthyliques puis par un enrichissement des oméga-3 par distillation moléculaire et/ou co-cristallisation des acides gras saturés et mono-insaturés à l'urée. Les esters éthyliques enrichis sont éventuellement reconvertis en triglycérides par voie chimique ou de préférence par voie enzymatique.

Cependant, ces procédés de séparation ne sont pas satisfaisants pour la production d'un oméga-3 tel que l'EPA, le DHA ou encore l'acide stéaridonique (SDA, C18-Sw3) à plus de 80 %, voire encore à plus de 96 %, notamment sous forme estérifiée.

Or, la purification des oméga-3 est délicate car ces composés comprennent plusieurs doubles liaisons carbone-carbone qui les rendent sensible à l'oxydation ou la dégradation. En présence d'oxygène et lorsqu'ils sont chauffés, ces PUFA subissent notamment des réactions d'isomérisation, d'oxydation, de peroxydation et d'oligomérisation.

Ainsi, les techniques de séparation énoncées ci-dessus permettent d'obtenir un mélange de PUFA avec un bon rendement et un degré de pureté acceptable ; mais elles ne peuvent pas être mises en œuvre pour la séparation individuelle des PUFA. Elles ne permettent donc pas de séparer des oméga-3 entre eux. En effet, la distillation moléculaire, par exemple, ne peut pas économiquement éliminer le DHA de l'EPA ou du SDA ; elle ne permet pas une séparation efficace des oméga-3 à longue chaîne de type C20 et C22. La combinaison de la clathration à l'urée et de la distillation moléculaire permet d'obtenir des mélanges d'oméga-3 à plus haute pureté, au prix d'un rendement généralement faible et d'un coût opératoire élevé, mais ne peut pas être utilisée pour la séparation des oméga-3 à longue chaîne entre eux, et de l'EPA et du DHA en particulier.

Il existe donc un besoin de fournir un procédé de purification industriel d'oméga-3 sous forme estérifiée à très haute pureté.

La chromatographie est une technique de séparation fine permettant la purification ou l'enrichissement efficace de molécules dans des conditions douces et à l'abri de la lumière et de l'air.

Cette technologie repose sur la séparation des molécules qui sont mises en contact avec une phase stationnaire avec lesquelles elles ont des interactions différentes. L'utilisation d'un ou plusieurs fluides, dits phases mobiles ou éluants, permet la percolation des différentes molécules à différentes vitesses. Ces différentes vitesses permettent de séparer physiquement les molécules et de les récolter sous forme purifiée à l'issue de procédés chromatographiques à une ou plusieurs colonnes. Les fractions purifiées sont en général concentrées, dans des conditions douces à température ambiante ou modérée, par des moyens tels que l'évaporation sous vide ou les procédés membranaires.

Dans certains cas, le produit de départ de la purification chromatographique est une huile composée d'esters d'acides gras déjà enrichie par distillation moléculaire, comprenant de préférence plus de 30 % de l'oméga-3 d'intérêt, qui a subi un traitement d'élimination des composés oxydés, soit par la dernière distillation moléculaire, soit par adsorption, de préférence sur des dérivés de silice (gel de silice, bentonite, terre de diatomée) ou sur charbon actif par exemple.

Un certain nombre de procédés chromatographiques ont été décrits, pour l'obtention d'oméga-3 avec une haute pureté.

Ainsi, le document US 5,719,302 décrit un procédé dans lequel les PUFA sont séparés notamment à l'aide d'un éluant supercritique (le gaz carbonique sous pression), et notamment sur lit mobile simulé (SMB pour « Simulated Moving Bed » selon la terminologie anglo-saxonne).

Le document US 2011/0091947 décrit un autre procédé de purification d'oméga-3 utilisant la technique de la chromatographie en lit mobile simulé. Le document décrit en particulier la succession d'une étape de transestérification enzymatique, de deux étapes de distillation moléculaire, et d'une étape de type SMB, ces trois dernières étapes permettent de séparer les produits en deux fractions par ordre de temps de rétention.

Le document WO 2011/080503 décrit la purification d'oméga-3 à partir d'un dispositif comprenant deux dispositifs chromatographiques SMB disposé en série et une zone de lavage, chaque dispositif chromatographique SMB définissant une zone de séparation et étant constitué de plusieurs colonnes. La charge à traiter est injectée dans une première zone de séparation pour obtenir un flux d'extrait et un flux de raffinat, ledit flux de raffinat comprenant les composés d'intérêts étant ensuite injecté dans une colonne de la seconde zone de séparation non adjacente à une colonne de la première zone.

Le document WO 2013/005051 décrit la purification d'oméga-3 par deux séparations chromatographiques par SMB ou AMB (« Actual Moving Bed », c'est-à-dire lit mobile réel) en phase inversée avec un éluant hydro-organique, dans lequel les deux séparations par SMB ou AMB sont réalisées séquentiellement sur le même dispositif chromatographique, ou sur deux dispositifs différents, l'intermédiaire purifié par le premier dispositif étant introduit dans le second.

Le document WO 2013/005048 décrit la purification d'EPA à plus de 90 % de pureté par une première séparation chromatographique suivie de deux séparations chromatographiques par SMB ou AMB en phase inversée avec un éluant hydro-organique à chaque étape, l'intermédiaire purifié par la première séparation chromatographique étant introduit dans la seconde séparation chromatographique, et l'intermédiaire purifié par la deuxième séparation chromatographique étant introduit dans la troisième séparation chromatographique.

Il existe encore un besoin de fournir un procédé de purification d'acide gras, et notamment d'acide gras polyinsaturé, pouvant être mis en œuvre dans une installation chromatographique plus simple que celles de l'état de la technique, avec par ailleurs une productivité spécifique (masse de produit purifié par masse de phase stationnaire et par unité de temps) élevée et une faible consommation de solvants, de sorte à réduire les coûts d'investissement.

### RESUME DE L'INVENTION

L'invention concerne en premier lieu un procédé de purification d'un premier acide gras à partir d'un mélange initial comprenant en outre au moins un deuxième acide gras, optionnellement un troisième acide gras et optionnellement un quatrième acide gras, le procédé comprenant :
- une première étape de séparation chromatographique en phase liquide, à partir du mélange initial, effectuée dans une première unité de séparation chromatographique, permettant de récupérer d'une part un premier flux enrichi en premier acide gras et d'autre part un flux enrichi en deuxième acide gras,
- une deuxième étape de séparation chromatographique en phase liquide, à partir du premier flux enrichi en premier acide gras, effectuée dans une deuxième unité de séparation chromatographique, permettant de récupérer d'une part un deuxième flux enrichi en premier acide gras et d'autre part un flux enrichi en troisième acide gras ;
- optionnellement, une troisième étape de séparation chromatographique en phase liquide, à partir du troisième flux enrichi en premier acide gras, effectuée dans une troisième unité de séparation chromatographique, permettant de récupérer d'une part un troisième flux enrichi en premier acide gras et d'autre part un flux enrichi en quatrième acide gras ;
au moins l'une parmi la première unité de séparation chromatographique, la deuxième unité de séparation chromatographique et la troisième unité de séparation chromatographique étant une unité de séparation chromatographique à lit statique à colonne unique avec recyclage à l'état stationnaire.

Selon un mode de réalisation, la première unité de séparation chromatographique est une unité de séparation chromatographique à lit statique à colonne unique avec recyclage à l'état stationnaire ; et/ou la deuxième unité de séparation chromatographique est une unité de séparation chromatographique à lit statique à colonne unique avec recyclage à l'état stationnaire ; et/ou la troisième unité de séparation chromatographique est une unité de séparation chromatographique à lit statique à colonne unique avec recyclage à l'état stationnaire.

Selon un mode de réalisation, au moins deux unités parmi la première unité de séparation chromatographique, la deuxième unité de séparation chromatographique et la troisième unité de séparation chromatographique sont des unités de séparation chromatographique à lit statique à colonne unique avec recyclage à l'état stationnaire.

Selon un mode de réalisation, au moins l'une parmi la première unité de séparation chromatographique, la deuxième unité de séparation chromatographique et la troisième unité de séparation chromatographique, et de préférence la première unité de séparation chromatographique, est une unité de séparation chromatographique à plusieurs colonnes ; et de manière plus particulièrement préférée est un système à lit mobile simulé ou à lit mobile réel ou un système dans lequel les points d'injection et les points de collecte des flux sont déplacés périodiquement de manière asynchrone.

Selon un mode de réalisation :
- la première étape de séparation chromatographique est mise en œuvre avec un premier éluant qui est un éluant hydro-organique ; et/ou
- la deuxième étape de séparation chromatographique est mise en œuvre avec un deuxième éluant qui est un éluant hydro-organique ; et/ou
- le cas échéant, la troisième étape de séparation chromatographique est mise en œuvre avec un troisième éluant qui est un éluant hydro-organique.

Selon un mode de réalisation :
- le premier éluant est un mélange cétone / eau, de manière plus particulièrement préférée acétone / eau ;
- de préférence, le deuxième éluant est un mélange alcool / eau, de manière plus particulièrement préférée méthanol / eau ;
- le cas échéant, de préférence, le troisième éluant est un mélange cétone / eau, de manière plus particulièrement préférée acétone / eau ;
- le cas échéant, de préférence le premier éluant est différent du deuxième éluant et le cas échéant, de préférence, le troisième éluant est différent du premier éluant et du deuxième éluant.

Selon un mode de réalisation :
- la concentration massique du premier éluant en solvant(s) organique(s) est contrôlée à 2 % près, de préférence à 1 % près, ou à 0,5 % près, ou à 0,2 % près, ou à 0,1 % près ;
- de préférence, la concentration massique du deuxième éluant en solvant(s) organique(s) est contrôlée à 2 % près, de préférence à 1 % près, ou à 0,5 % près, ou à 0,2 % près, ou à 0,1 % près ;
- le cas échéant, de préférence, la concentration massique du troisième éluant en solvant(s) organique(s) est contrôlée à 2 % près, de préférence à 1 % près, ou à 0,5 % près, ou à 0,2 % près, ou à 0,1 % près.

Selon l'invention, le premier acide gras est un premier acide gras polyinsaturé.

Selon des exemples :
- le premier acide gras polyinsaturé est l'acide eicosapentaénoïque, et est de préférence récupéré à l'issue du procédé avec une pureté supérieure ou égale à 80 %, ou à 90 %, ou à 96 % ; ou
- le premier acide gras polyinsaturé est l'acide docosahexaénoïque, et est de préférence récupéré à l'issue du procédé avec une pureté supérieure ou égale à 70 %, ou à 80 %, ou à 90 %, ou à 95 % ; ou
- le premier acide gras polyinsaturé est l'acide arachidonique, et est de préférence récupéré à l'issue du procédé avec une pureté supérieure ou égale à 70 %, ou à 80 %, ou à 90 %, ou à 95 % ; ou
- le premier acide gras polyinsaturé est l'acide docosapentaénoïque, et est de préférence récupéré à l'issue du procédé avec une pureté supérieure ou égale à 70 %, ou à 80 %, ou à 90 %, ou à 95 %.

Selon un mode de réalisation, le procédé comprend :
- au moins une étape de séparation chromatographique entre le premier acide gras et un ou des composés plus retenus que celui-ci, et de préférence au moins deux étapes de séparation chromatographique entre le premier acide gras et un ou des composés plus retenus que celui-ci ; et/ou
- au moins une étape de séparation chromatographique entre le premier acide gras et un ou des composés moins retenus que celui-ci ; et
le procédé comprenant de préférence :
- la première étape de séparation chromatographique qui est une étape de séparation chromatographique entre le premier acide gras et un ou des composés plus retenus que celui-ci, puis la deuxième étape de séparation chromatographique qui est une étape de séparation chromatographique entre le premier acide gras et un ou des composés plus retenus que celui-ci, puis la troisième étape de séparation chromatographique qui est une étape de séparation chromatographique entre le premier acide gras et un ou des composés moins retenus que celui-ci.

Selon un mode de réalisation, au moins l'une parmi la première unité de séparation chromatographique, la deuxième unité de séparation chromatographique et la troisième de séparation chromatographique comprend une colonne de séparation présentant une longueur supérieure ou égale à 5 cm, ou à 10 cm, ou à 20 cm, ou à 25 cm, ou à 30 cm, ou à 40 cm, ou à 50 cm, ou à 60 cm ; et/ou présentant un diamètre supérieur ou égal à 10 cm, ou à 20 cm, ou à 25 cm, ou à 30 cm, ou à 40 cm, ou à 50 cm, ou à 60 cm.

Selon un mode de réalisation :
- le mélange initial qui alimente la première unité de séparation chromatographique comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le premier flux enrichi en premier acide gras qui alimente la deuxième unité de séparation chromatographique comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le cas échéant, le deuxième flux enrichi en premier acide gras qui alimente la troisième unité de séparation chromatographique comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques.

L'invention a également pour objet une installation de purification d'un premier acide gras à partir d'un mélange initial, l'installation comprenant :
- une première unité de séparation chromatographique en phase liquide, alimentée par une conduite d'amenée de mélange initial, et à laquelle sont connectées en sortie d'une part une première conduite de flux enrichi en premier acide gras et d'autre part une conduite de flux enrichi en deuxième acide gras ;
- une deuxième unité de séparation chromatographique en phase liquide, alimentée par la première conduite de flux enrichi en premier acide gras, et à laquelle sont connectées en sortie d'une part une deuxième conduite de flux enrichi en premier acide gras et d'autre part une conduite de flux enrichi en troisième acide gras
- optionnellement, une troisième unité de séparation chromatographique en phase liquide, alimentée par la deuxième conduite de flux enrichi en premier acide gras, et à laquelle sont connectées en sortie d'une part une troisième conduite de flux enrichi en premier acide gras et d'autre part une conduite de flux enrichi en quatrième acide gras ;
dans laquelle au moins l'une parmi la deuxième unité de séparation chromatographique et la troisième unité de séparation chromatographique est une unité de séparation chromatographique à lit statique à colonne unique avec recyclage à l'état stationnaire.

Selon un mode de réalisation, la première unité de séparation chromatographique est une unité de séparation chromatographique à lit statique à colonne unique avec recyclage à l'état stationnaire ; et la deuxième unité de séparation chromatographique est une unité de séparation chromatographique à lit statique à colonne unique avec recyclage à l'état stationnaire ; et/ou la troisième unité de séparation chromatographique est une unité de séparation chromatographique à lit statique à colonne unique avec recyclage à l'état stationnaire ; et de préférence au moins deux unités parmi la première unité de séparation chromatographique, la deuxième unité de séparation chromatographique et la troisième unité de séparation chromatographique sont des unités de séparation chromatographique à lit statique à colonne unique avec recyclage à l'état stationnaire.

Selon un mode de réalisation, au moins l'une parmi la première unité de séparation chromatographique, la deuxième unité de séparation chromatographique et la troisième unité de séparation chromatographique, et de préférence la première unité de séparation chromatographique, est une unité de séparation chromatographique à plusieurs colonnes ; et de manière plus particulièrement préférée est un système à lit mobile simulé ou à lit mobile réel ou un système dans lequel les points d'injection et les points de collecte des flux sont déplacés périodiquement de manière asynchrone.

Selon un mode de réalisation, au moins l'une parmi la première unité de séparation chromatographique, la deuxième unité de séparation chromatographique et la troisième unité de séparation chromatographique comprend une colonne de séparation présentant une longueur supérieure ou égale à 5 cm, ou à 10 cm, ou à 20 cm, ou à 25 cm, ou à 30 cm, ou à 40 cm, ou à 50 cm, ou à 60 cm ; et/ou présentant un diamètre supérieur ou égal à 10 cm, ou à 20 cm, ou à 25 cm, ou à 30 cm, ou à 40 cm, ou à 50 cm, ou à 60 cm.

La présente invention permet de surmonter les inconvénients de l'état de la technique. Elle fournit plus particulièrement un procédé de purification d'acide gras, et notamment d'acide gras polyinsaturé, pouvant être mis en œuvre dans une installation chromatographique plus simple que celles de l'état de la technique, avec par ailleurs une productivité spécifique élevée et une faible consommation de solvants.

Cela est accompli grâce à la mise en œuvre d'une étape de séparation dans une unité de séparation à lit statique mono-colonne avec recyclage à l'état stationnaire.

L'invention permet ainsi de fournir un acide gras, notamment acide gras polyinsaturé, de pureté élevée, permettant son utilisation dans des compositions de suppléments alimentaires ou de médicaments, à partir d'une charge multi-composés, et ce en minimisant le nombre de colonnes chromatographiques employées.

### BREVE DESCRIPTION DES FIGURES

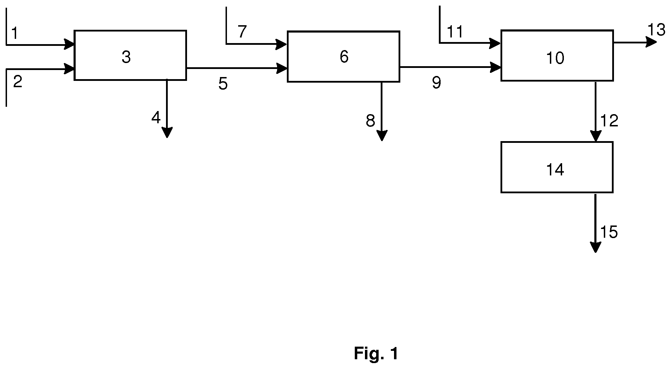

La **figure 1** représente de manière schématique un mode de réalisation d'une installation pour la mise en œuvre de l'invention.

### DESCRIPTION DE MODES DE REALISATION DE L'INVENTION

L'invention est maintenant décrite plus en détail et de façon non limitative dans la description qui suit.

De manière générale, les proportions exprimées sont des proportions massiques, sauf mention contraire.

L'ensemble de la description qui suit est effectuée en relation avec le mode de réalisation préféré dans lequel le premier acide gras est un PUFA, appelé « *premier PUFA* ». Toutefois, cette description vaut de manière analogue lorsque le premier acide gras n'est pas polyinsaturé. Il convient alors de remplacer « *premier PUFA* » par le terme plus général « *premier acide gras* » dans la description ci-dessous. Ainsi, le premier acide gras peut également être un acide gras saturé, un acide gras mono-insaturé, ou un dérivé d'acide gras tel qu'un acide gras ramifié, naturel ou modifié.

### Procédé de préparation

Le procédé de l'invention permet d'obtenir un premier PUFA sous forme purifiée, à partir d'un mélange initial. Le mélange initial comprend, outre le premier PUFA, d'autres acides gras non souhaités, à savoir généralement des acides gras saturés ou mono-insaturés et d'autres PUFA, ainsi que d'autres impuretés éventuelles. Le deuxième acide gras, le troisième acide gras et le quatrième acide gras, mentionnés ci-dessus, font partie de ceux-ci.

Le mélange initial peut être un mélange d'acides gras dérivé de poisson, de végétaux, d'algues et/ou de levure, et de préférence de poisson. Il peut s'agir d'une matière brute, par exemple de l'huile de poisson ou de l'huile d'algue ou de l'huile de levure. Il peut également s'agir d'un produit dérivé des matières brutes ci-dessus, et par exemple dérivé d'huile de poisson, d'huile d'algue et/ou d'huile de levure. L'huile peut par exemple être extraite de végétaux, algues ou levures naturelles ou génétiquement modifiées.

Par « *produit dérivé d'une matière brute* », on entend une matière brute qui a été soumise à une ou plusieurs étapes de traitement. Ces étapes de traitement peuvent comprendre une ou plusieurs étapes de désintégration cellulaire, de broyage, de séparation ou de purification (par exemple un fractionnement) et/ou une étape d'hydrolyse pour convertir des triglycérides en acides gras libres et/ou une étape de transestérification pour convertir les acides gras en alkyl esters, et de préférence en éthyl esters, et/ou une étape de réduction de l'indice de peroxyde et/ou de l'indice d'anisidine (cf. ci-dessous), et/ou une étape de distillation moléculaire, et/ou une ou plusieurs étapes de séparation chromatographique, etc.

Selon un mode de réalisation avantageux, le mélange initial est un produit estérifié ou transestérifié, tel qu'une huile de poisson, une huile végétale, une huile d'algue ou une huile de levure transestérifiée.

Ainsi, chaque acide gras (et en particulier chaque PUFA) obtenu ou utilisé dans le procédé de l'invention peut être un dérivé d'acide gras, notamment sous la forme d'un monoglycéride, diglycéride ou triglycéride, d'un ester, d'un phospholipide, d'un amide, d'une lactone ou d'un sel.

Les formes acide gras libre et esters sont préférées, et tout particulièrement les esters. Les esters sont typiquement des alkyl esters, par exemple des alkyl esters en C1-C6, notamment en C1-C4, par exemple des méthyl esters et des éthyl esters. Les éthyl esters sont préférés.

Ainsi, le premier PUFA, le deuxième acide gras, le troisième acide gras et le quatrième acide gras mentionnés dans la présente demande peuvent être par exemple sous forme acide gras libre ou ester, et de préférence sont sous forme de composés éthyl esters.

En faisant référence à la **figure 1****,** le procédé selon l'invention peut être mis en œuvre dans une installation comprenant une première unité de séparation chromatographique 3. La première unité de séparation chromatographique 3 assure la séparation entre le premier PUFA et le deuxième acide gras.

A chaque fois qu'il est fait mention dans la présente demande d'une séparation entre le premier PUFA et un acide gras donné, il est entendu que d'autres acides gras peuvent également être séparés du premier PUFA simultanément avec la séparation vis-à-vis de l'acide gras donné.

Généralement, chaque séparation chromatographique sépare le premier PUFA d'un ensemble de composés plus polaires que lui ou moins polaires que lui. La séparation peut également s'effectuer selon des critères de taille de longueur de chaine aliphatique et de nombre d'insaturation. Plus généralement, les effets pouvant être dépendants des éluants utilisés, la séparation s'effectue selon des critères de temps de rétention qui sont différents selon le cas, permettant ainsi de séparer le premier PUFA d'impuretés qui sont plus ou moins retenues que lui.

La température de la séparation peut être ajustée selon les critères bien connus de l'homme de l'art dans une gamme située entre 5°C et 90°C, préférentiellement entre 15 et 60°C, et encore préférentiellement entre la température ambiante et 45°C. Le cas échéant la pression est ajustée pour maintenir un état monophasique, de préférence liquide ou supercritique dans la colonne.

La première unité de séparation chromatographique 3 est alimentée par une conduite d'alimentation en mélange d'acides gras 1 ainsi que par une conduite d'alimentation en premier éluant 2.

En sortie de la première unité de séparation chromatographique 3 sont connectées d'une part une première conduite de flux enrichi en premier
PUFA 5 et d'autre part une conduite de flux enrichi en deuxième acide gras 4.

Dans le contexte de la présente demande, le terme « *enrichi* » a une signification relative : une séparation entre une espèce A et une espèce B à partir d'un flux initial, permettant de récupérer un flux enrichi en espèce A, signifie ainsi que le flux ainsi récupéré présente un rapport massique A/B supérieur à celui du flux initial.

Le mélange initial peut avoir subi des étapes préliminaires de traitement telles que décrites ci-dessus, auquel cas les unités de traitement correspondantes, non représentées, peuvent être incluses dans l'installation de l'invention.

Selon l'invention, une deuxième unité de séparation chromatographique 6 est prévue en aval, pour assurer une séparation entre le premier PUFA et un troisième acide gras. Cette deuxième unité de séparation chromatographique 6 est alimentée par la première conduite de flux enrichi en premier PUFA 5 ainsi que par une conduite d'alimentation en deuxième éluant 7.

En sortie de la deuxième unité de séparation chromatographique 6 sont connectées d'une part une deuxième conduite de flux enrichi en premier PUFA 9 et d'autre part une conduite de flux enrichi en troisième acide gras 8.

De préférence, une troisième unité de séparation chromatographique 10 est prévue, pour assurer une séparation entre le premier PUFA et un quatrième acide gras. Cette troisième unité de séparation chromatographique 10 est alimentée par la deuxième conduite de flux enrichi en premier PUFA 9 ainsi que par une conduite d'alimentation en troisième éluant 11.

En sortie de la troisième unité de séparation chromatographique 10 sont connectées d'une part une troisième conduite de flux enrichi en premier PUFA 12 et d'autre part une conduite de flux enrichi en quatrième acide gras 13.

De préférence, le procédé comporte exactement trois étapes de séparation chromatographique dans les trois unités décrites ci-dessus.

Alternativement, le procédé ne comprend que deux séparations chromatographiques, auquel cas la troisième unité de séparation chromatographique 10 est omise. Le premier PUFA sous forme purifiée est donc directement récupéré dans la deuxième conduite de flux enrichi en premier PUFA 9.

Alternativement encore, le procédé peut comprendre quatre étapes de séparation chromatographique successives (ou davantage), auquel cas des unités de séparation chromatographique supplémentaires sont ajoutées de manière analogue.

Le terme « *unité de séparation chromatographique* » désigne soit un système chromatographique à colonne unique soit un système chromatographique à plusieurs colonnes.

Il peut s'agir d'un système chromatographique à lit statique ou non. Dans un système chromatographique à lit statique, le mélange de composés à séparer percole dans une enceinte (ou colonne), généralement cylindrique. La colonne contient un lit de matériau poreux (phase stationnaire) perméable aux fluides. La vitesse de percolation de chaque composé dans le mélange dépend des propriétés physiques du composé. Les composés les plus retenus sur la phase stationnaire percolent plus lentement que les composés les moins retenus sur la phase stationnaire. Ce principe permet d'effectuer la séparation souhaitée.

Il est possible d'effectuer un tel traitement dans plusieurs colonnes en série ou en parallèle, mais généralement une séparation chromatographique dans un système à lit statique est mise en œuvre avec une colonne unique.

Des exemples de tels systèmes chromatographiques à lit statique sont les systèmes HPLC (chromatographie en phase liquide à haute performance) ou CYCLOJET™ (c'est-à-dire système avec recyclage à l'état stationnaire).

Le système CYCLOJET™ est tel que décrit dans le document US 6,063,284, auquel il est fait expressément référence. Il s'agit d'un système de séparation chromatographique discontinue à colonne unique, dans lequel les espèces (i) les plus retenues puis (ii) les moins retenues sont collectées séparément à la sortie de la colonne, une portion non-séparée du chromatogramme étant recyclée par une pompe principale. Le mélange à séparer est périodiquement injecté au moyen d'une boucle d'injection dans la portion recyclée du chromatogramme. La boucle d'injection est de préférence connectée entre la pompe principale et la colonne. Après plusieurs cycles chromatographiques, le procédé atteint un état stationnaire périodique dans lequel la quantité de produits injectés est égale à la quantité de produits collectés séparément à la sortie de la colonne.

Selon un mode de réalisation, la séparation chromatographique dans un système à lit statique mono-colonne avec recyclage à l'état stationnaire est cyclique et comprend les étapes suivantes :
- établissement et maintien d'un profil chromatographique circulant dans la colonne au moyen d'une pompe à éluant ;
- injection dans ledit profil chromatographique circulant d'un échantillon comprenant les au moins deux composés à séparer, de manière discontinue et à chaque cycle, l'injection étant effectuée au moyen d'une boucle d'injection contrôlée dans une position d'injection par une vanne d'injection, afin d'injecter l'échantillon présent dans la boucle dans le profil chromatographique circulant, la vanne d'injection restant en position d'injection du début de l'injection jusqu'au moment où la totalité du profil est élué de la colonne, puis basculement de la vanne d'injection dans une position de charge, pour charger la boucle d'injection lorsque tout le profil est dans la colonne, et
- collecte d'au moins deux fractions enrichies à partir du profil circulant, de manière discontinue et périodique.

Cette séparation peut également comprendre l'étape suivante :
- passage d'éluant dans la colonne en tant que phase mobile, de manière essentiellement continue au cours du cycle, au moyen de la pompe à éluant.

Cette séparation peut également comprendre les étapes suivantes :
- enregistrement des événements se produisant à partir du début de la collecte d'une première fraction jusqu'au début suivant de collecte de première fraction ;
- interruption de la pompe à éluant lors de la collecte d'une troisième fraction, cette interruption se poursuivant jusqu'à la fin du cycle, de sorte que les cycles soient reproductibles temporellement.

Selon un mode de réalisation, il n'y a aucune perte de profil circulant lors de l'injection dans le profil circulant maintenu.

Un mode de réalisation détaillé de ce système figure en col.5 I.36-col.10 I.41 du document US 6,063,284 précité.

L'unité de séparation chromatographique peut également être un système chromatographique à lit non-statique. Un système à lit non-statique est un système multi-colonnes, dans lequel les positions relatives du lit de phase stationnaire et des points d'injection et/ou de collecte des flux se déplacent au cours du temps.

Des exemples de tels systèmes chromatographiques à lit non-statique sont les systèmes SMB, iSMB, SSMB, AMB, VARICOL™, MODICON™, POWERFEED™, DCC ou MCSGP.

Un système SMB comprend une pluralité de colonnes individuelles contenant un adsorbant, qui sont connectées en série. Un flux d'éluant traverse les colonnes selon une première direction. Les points d'injection du flux d'alimentation et de l'éluant, ainsi que les points de collecte des composés séparés, sont décalés périodiquement et simultanément au moyen d'un ensemble de vannes. L'effet global est de simuler le fonctionnement d'une colonne unique contenant un lit mobile d'adsorbant solide, l'adsorbant solide se déplaçant dans une direction à contre-courant du flux d'éluant. Ainsi, un système SMB est composé de colonnes qui contiennent des lits stationnaires d'adsorbant solide à travers lesquels passe l'éluant, mais le fonctionnement est tel qu'un lit mobile continu à contre-courant est simulé.

La forme la plus conventionnelle d'un système SMB est le système SMB à quatre zones. D'autres formes possibles sont les systèmes SMB à trois zones et les systèmes SMB à deux zones (tels que décrits dans l'article « Two Section Simulated Moving Bed Process » de Kwangnam Lee, dans Séparation Science and Technology 35(4):519-534, 2000, auquel il est fait expressément référence).

Un système iSMB est tel que décrit dans les documents EP 0342629 et US 5,064,539, auxquels il est fait expressément référence. Un système SSMB découpe les introductions et collectes des flux en sous séquences appliquées de façons périodiques. Dans les systèmes iSMB et SSMB, il y a au moins une étape dans laquelle le système fonctionne en boucle fermée, sans entrée ou sortie de produit.

D'autres variantes des systèmes SMB sont : le système SMB variant dans le temps et le système POWERFEED™, tels que décrits dans le document US 5,102,553 et dans l'article « PowerFeed opération of simulated moving bed units: changing flow-rates during the switching interval », de Zhang et al. dans Journal of Chromatography A, 1006:87-99, 2003, auxquels il est fait expressément référence; le système MODICON™, tel que décrit dans le document US 7,479,228, auquel il est fait expressément référence ; et le système SMB avec recirculation interne, tel que décrit dans le document US 8,282,831, auquel il est fait expressément référence.

Un système de chromatographie DCC est tel que décrit dans le document FR 2889077, auquel il est fait expressément référence. Un système DCC est un procédé séquentiel à déplacement périodique des points d'injection de phase mobile et de mélange à séparer, ayant la caractéristique d'être constamment en boucle ouverte. Il utilise deux colonnes ou plus.

Un système AMB présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

Un système de chromatographie VARICOL™ est tel que décrit dans les documents US 6,136,198, US 6,375,839 US 6,413,419 et US 6,712,973, auxquels il est fait expressément référence. Un système VARICOL™ comprend une pluralité de colonnes individuelles contenant un adsorbant qui sont reliées en série. On fait passer un éluant dans les colonnes selon une première direction. Contrairement au système SMB, les points d'injection pour le mélange à séparer et pour l'éluant et les points de collecte des composés séparés dans le système sont déplacés périodiquement mais de manière asynchrone, au moyen d'un ensemble de vannes. L'effet global est de créer des zones de séparation de longueur variable dans le temps, allouant ainsi la phase stationnaire de manière dynamique dans les zones où elle est la plus utile, et permettant une puissance de séparation similaire avec moins d'unités de séparation chromatographique et une productivité accrue. Contrairement à un système SMB, un système VARICOL™ ne simule pas le fonctionnement d'une colonne unique contenant un lit mobile d'adsorbant solide, l'adsorbant solide se déplaçant dans une direction à contre-courant du flux d'éluant, et ainsi le principe de fonctionnement du VARICOL™ ne peut pas être mis en œuvre dans un système AMB équivalent.

L'invention prévoit qu'au moins une étape de séparation chromatographique est effectuée dans une unité à lit statique mono-colonne avec recyclage à l'état stationnaire (notamment unité CYCLOJET™ telle que décrite ci-dessus).

Lorsque le procédé comporte uniquement deux étapes de séparation chromatographique, successivement dans la première unité de séparation chromatographique 3 et dans la deuxième unité de séparation chromatographique 6, alors :
- soit uniquement la première unité de séparation chromatographique 3 est une unité à lit statique mono-colonne avec recyclage à l'état stationnaire ;
- soit uniquement la deuxième unité de séparation chromatographique 6 est une unité à lit statique mono-colonne avec recyclage à l'état stationnaire ;
- soit à la fois la première unité de séparation chromatographique 3 et la deuxième unité de séparation chromatographique 6 sont des unités à lit statique mono-colonnes avec recyclage à l'état stationnaire.

Lorsque le procédé comporte trois étapes de séparation chromatographique, successivement dans la première unité de séparation chromatographique 3, la deuxième unité de séparation chromatographique 6 et la troisième unité de séparation chromatographique 10, alors :
- soit uniquement la première unité de séparation chromatographique 3 est une unité à lit statique mono-colonne avec recyclage à l'état stationnaire ;
- soit uniquement la deuxième unité de séparation chromatographique 6 est une unité à lit statique mono-colonne avec recyclage à l'état stationnaire ;
- soit uniquement la troisième unité de séparation chromatographique 10 est une unité à lit statique mono-colonne avec recyclage à l'état stationnaire ;
- soit la première unité de séparation chromatographique 3 et la deuxième unité de séparation chromatographique 6 uniquement sont des unités à lit statique mono-colonnes avec recyclage à l'état stationnaire ;
- soit la première unité de séparation chromatographique 3 et la troisième unité de séparation chromatographique 10 uniquement sont des unités à lit statique mono-colonnes avec recyclage à l'état stationnaire ;
- soit la deuxième unité de séparation chromatographique 6 et la troisième unité de séparation chromatographique 10 uniquement sont des unités à lit statique mono-colonnes avec recyclage à l'état stationnaire ;
- soit à la fois la première unité de séparation chromatographique 3, la deuxième unité de séparation chromatographique 6 et la troisième unité de séparation chromatographique 10 sont des unités à lit statique mono-colonnes avec recyclage à l'état stationnaire.

Les unités de séparation chromatographique qui ne sont pas des unités à lit statique avec recyclage à l'état stationnaire peuvent être des unités de séparation à lit statique, mono-colonnes ou non, ou des unités de séparation à lit non-statique (multi-colonnes), notamment du type VARICOL™ ou SMB ou AMB.

Ainsi, selon un mode de réalisation, la première étape est mise en œuvre dans une unité à lit non-statique, la deuxième étape est mise en œuvre dans une unité à lit statique, et le cas échéant la troisième étape est mise en œuvre dans une unité à lit statique.

L'expression « *le cas échéant»* signifie « *dans l'hypothèse où l'étape de séparation chromatographique en question est effectivement présente ».*

Selon un mode de réalisation, la première étape est mise en œuvre dans une unité VARICOL™ ou SMB ou AMB, la deuxième étape est mise en œuvre dans une unité CYCLOJET™, et la troisième est mise en œuvre dans une unité VARICOL™ ou SMB ou AMB.

Selon un mode de réalisation alternatif et préféré, la première étape est mise en œuvre dans une unité VARICOL™ ou SMB ou AMB, la deuxième étape est mise en œuvre dans une unité HPLC ou CYCLOJET™, et la troisième est mise en œuvre dans une unité HPLC ou CYCLOJET™ (l'une au moins de ces deux dernières étapes étant mise en œuvre dans une unité CYCLOJET™).

Dans une variante préférée, la première étape est mise en œuvre dans une unité VARICOL™, la deuxième étape est mise en œuvre dans une unité CYCLOJET™, et la troisième est mise en œuvre dans une unité CYCLOJET™.

Selon une autre variante, chacune des étapes est mise en œuvre dans une unité HPLC ou CYCLOJET™, étant précisé que l'une au moins des étapes est mise en œuvre dans une unité CYCLOJET™ ; de préférence deux des étapes sont mises en œuvre dans une unité CYCLOJET™ ; et éventuellement les trois étapes sont mises en œuvre dans une unité CYCLOJE T™.

Les étapes de séparation peuvent être effectuées simultanément dans des unités physiquement distinctes, ou peuvent être effectuées séquentiellement, dans des unités physiquement distinctes ou dans des mêmes unités.

Il faut noter que lorsque deux étapes de séparation chromatographique sont effectuées dans un système de type SMB ou AMB, il est possible de les mettre en œuvre simultanément sur un même système SMB ou AMB. Un exemple de mise en œuvre simultanée sur un même appareil est décrit dans le document WO 2011/080503 ou le document WO 2013/005048, ou le document WO 2013/005051, auxquels il est fait expressément référence.

De préférence, toutes les unités de séparation chromatographique sont physiquement distinctes.

Selon un mode de réalisation, la ou les colonnes de séparation (de préférence les colonnes de séparation) de la première unité de séparation chromatographique 3 présentent une longueur totale ou cumulée supérieure ou égale à 5 cm, ou à 10 cm, ou à 20 cm, ou à 25 cm, ou à 30 cm, ou à 40 cm, ou à 50 cm, ou à 60 cm ; et/ou présentent un diamètre supérieur ou égal à 10 cm, ou à 20 cm, ou à 25 cm, ou à 30 cm, ou à 40 cm, ou à 50 cm, ou à 60 cm.

Selon un mode de réalisation, la ou les colonne de séparation (de préférence la colonne de séparation) de la deuxième unité de séparation chromatographique 6 présentent une longueur totale ou cumulée supérieure ou égale à 5 cm, ou à 10 cm, ou à 20 cm, ou à 25 cm, ou à 30 cm, ou à 40 cm, ou à 50 cm, ou à 60 cm ; et/ou présentent un diamètre supérieur ou égal à 10 cm, ou à 20 cm, ou à 25 cm, ou à 30 cm, ou à 40 cm, ou à 50 cm, ou à 60 cm.

Selon un mode de réalisation, la ou les colonne de séparation (de préférence la colonne de séparation) de la troisième unité de séparation chromatographique 10 présentent une longueur totale ou cumulée supérieure ou égale à 5 cm, ou à 10 cm, ou à 20 cm, ou à 25 cm, ou à 30 cm, ou à 40 cm, ou à 50 cm, ou à 60 cm ; et/ou présentent un diamètre supérieur ou égal à 10 cm, ou à 20 cm, ou à 25 cm, ou à 30 cm, ou à 40 cm, ou à 50 cm, ou à 60 cm.

Selon un mode de réalisation, la totalité des colonnes chromatographiques utilisées dans le procédé ou l'installation de l'invention présentent une longueur totale ou cumulée supérieure ou égale à 5 cm, ou à 10 cm, ou à 20 cm, ou à 25 cm, ou à 30 cm, ou à 40 cm, ou à 50 cm, ou à 60 cm ; et/ou présentent un diamètre supérieur ou égal à 10 cm, ou à 20 cm, ou à 25 cm, ou à 30 cm, ou à 40 cm, ou à 50 cm, ou à 60 cm.

La longueur et le diamètre des colonnes sont les dimensions utiles des colonnes, c'est-à-dire les dimensions du lit de phase stationnaire dans les colonnes. Différentes géométries de colonnes existent, les colonnes cylindriques axiales ou radiales mais également des cellules à section non cylindriques auxquelles il est fait expressément référence (dans ce cas, le diamètre fait référence à la dimension maximale de la section).

Chaque étape de séparation chromatographique peut être effectuée sur une phase inversée, en tant qu'adsorbant (phase stationnaire). Par exemple, on peut utiliser des adsorbants basés sur des résines faiblement polaires ou des phases stationnaires à base de silice chimiquement modifiée avec des groupements organiques tels que des groupements alkyles (notamment en C4, C8, C18, C24, C30), phényles, ou autres.

Chaque étape de séparation chromatographique peut être effectuée en utilisant un éluant hydro-organique, c'est-à-dire un mélange d'un ou plusieurs solvants organiques avec de l'eau. De préférence, toutes les étapes de séparation chromatographique sont effectuées en utilisant des éluants hydro-organiques. Alternativement, il est possible de mettre en œuvre certaines étapes de séparation chromatographiques avec des éluants purement organiques.

Les solvants organiques utilisables dans le cadre de l'invention (notamment pour former les éluants hydro-organiques) sont par exemple les alcools tels que l'éthanol, le propanol, l'isopropanol et de manière davantage préférée le méthanol ; les cétones telles que l'acétone ou la méthyléthyl-cétone ; les nitriles tels que l'acétonitrile ; les esters tels que l'acétate de méthyle ou l'acétate d'éthyle ; les furanes tels que le tétrahydrofurane ; les éthers tels que le diéthylether or le méthyléthylether ; et les combinaisons de deux ou plus de deux solvants parmi ceux-ci. Le méthanol et l'acétone sont les solvants organiques préférés.

Chaque éluant hydro-organique est caractérisé par un rapport eau/organique, qui est le rapport volumique de l'eau par rapport au(x) solvant(s) organique(s) dans l'éluant.

Le rapport eau/organique de chaque éluant hydro-organique peut de préférence varier de 0,01:99,99 à 30:70, et de préférence de 5:95 à 25:75.

Les différentes étapes de séparation chromatographique peuvent être effectuées avec des éluants ayant la même composition ou des compositions différentes.

Il est préféré d'utiliser des éluants ayant des compositions différentes, et en particulier ayant des rapports eau/organique différents, cela permettant d'ajuster la force éluante de l'éluant à chaque étape de séparation et donc d'obtenir la séparation de différents composés à chaque étape. On peut aussi souhaiter utiliser des éluants composés de différents solvants organiques dans les différentes étapes, afin d'ajuster la sélectivité chromatographique entre certaines espèces devant être séparées à chaque étape de séparation et ainsi obtenir la séparation de différents composés à chaque étape.

De préférence, la concentration massique du premier éluant en solvant(s) organique(s) est contrôlée à 2 % près, de préférence à 1 % près, ou à 0,5 % près, ou à 0,2 % près, ou à 0,1 % près ; de préférence, la concentration massique du deuxième éluant en solvant(s) organique(s) est contrôlée à 2 % près, de préférence à 1 % près, ou à 0,5 % près, ou à 0,2 % près, ou à 0,1 % près ; le cas échéant, de préférence, la concentration massique du troisième éluant en solvant(s) organique(s) est contrôlée à 2 % près, de préférence à 1 % près, ou à 0,5 % près, ou à 0,2 % près, ou à 0,1 % près. Le contrôle de la composition des éluants est effectué assurant des apports d'eau et/ou de solvant(s) organiques afin de procéder aux ajustements nécessaires.

Dans la première étape de séparation chromatographique, le premier PUFA est de préférence séparé de composés (notamment le deuxième acide gras) plus retenus que celui-ci. Dans ce cas, lorsque la première unité de séparation chromatographique 3 est une unité à lit non-statique, le flux enrichi en premier PUFA est le raffinat, et le flux enrichi en deuxième acide gras est l'extrait.

Dans la deuxième étape de séparation chromatographique, le premier PUFA est de préférence séparé de composés (notamment le troisième acide gras) plus retenus que celui-ci.

Dans la troisième étape de séparation chromatographique, le premier PUFA est de préférence séparé de composés (notamment le quatrième acide gras) moins retenus que celui-ci.

Chaque flux issu d'une étape de séparation chromatographique subit de préférence une étape de concentration. Les flux sont concentrés de sorte à en éliminer l'éluant (solvants organiques et eau) ou à réduire la teneur massique du flux en solvants organiques et eau à un niveau de moins de 90%, ou de moins de 80%, ou de moins de 70%, ou de moins de 60%, ou de moins de 50%, ou de moins de 40%, ou de moins de 30%, ou de moins de 20%, ou de moins de 10 %, ou de moins de 5 %, ou de moins de 2 %, ou de moins de 1 %, ou de moins de 0,5 %, ou de moins de 0,1 %.

Ainsi, dans un mode de réalisation préféré, au moins une unité de concentration (non représentée sur la figure) est associée à chaque unité de séparation chromatographique 3, 6, 10.

Ainsi, de préférence, le premier flux enrichi en premier PUFA collecté à l'issue de la première séparation chromatographique est un flux concentré (appauvri en éluant ou dépourvu ou essentiellement dépourvu de solvants organiques et d'eau) ; de même, le deuxième flux enrichi en premier PUFA collecté à l'issue de la deuxième séparation chromatographique est de préférence un flux concentré (appauvri en éluant ou dépourvu ou essentiellement dépourvu de solvants organiques et d'eau) ; de même, le cas échéant, le troisième flux enrichi en premier PUFA collecté à l'issue de la troisième séparation chromatographique est de préférence un flux concentré (appauvri en éluant ou dépourvu ou essentiellement dépourvu de solvants organiques et d'eau).

Eventuellement, le flux enrichi en deuxième acide gras, le cas échéant le flux enrichi en troisième acide gras et le cas échéant le flux enrichi en quatrième acide gras sont des flux concentrés (appauvris en éluant ou dépourvus ou essentiellement dépourvus de solvants organiques et d'eau).

Dans chaque unité de concentration, l'éluant peut être évaporé et condensé, de sorte à le séparer du mélange d'acides gras. On peut utiliser par exemple un évaporateur à film tombant à recirculation, un évaporateur à flux montant, un évaporateur à film raclé, un évaporateur à couche mince, un évaporateur à thermosiphon, un évaporateur rotatif, une colonne à distiller, une colonne de rectification ou tout autre évaporateur ou combinaison d'évaporateurs permettant l'évaporation de l'éluant et la concentration des acides gras concentrés au fond de l'appareil. L'évaporation est de préférence effectuée à une pression inférieure à la pression atmosphérique, notamment à une pression inférieure ou égale à 750 mbar, ou inférieure ou égale à 500 mbar, ou inférieure ou égale à 300 mbar.

Alternativement, on peut utiliser un dispositif de séparation membranaire, avec un ou plusieurs étages de séparation, ou une combinaison de moyens d'évaporation et de séparation membranaire.

L'éluant obtenu à l'issue d'une étape de concentration (par exemple évaporé et condensé, ou autrement séparé), peut être recyclé vers une ou plusieurs étapes du procédé, notamment une ou plusieurs des étapes de séparation chromatographique.

Ainsi, de préférence le procédé de l'invention prévoit un recyclage de l'éluant utilisé dans la première étape de séparation chromatographique. De manière plus particulièrement préférée, l'éluant est recyclé pour être réutilisé dans la première étape de séparation chromatographique.

De préférence le procédé de l'invention prévoit un recyclage de l'éluant utilisé dans la deuxième étape de séparation chromatographique. De manière plus particulièrement préférée, l'éluant est recyclé pour être réutilisé dans la deuxième étape de séparation chromatographique.

Ainsi, de préférence le procédé de l'invention prévoit un recyclage de l'éluant utilisé dans la troisième étape de séparation chromatographique. De manière plus particulièrement préférée, l'éluant est recyclé pour être réutilisé dans la troisième étape de séparation chromatographique.

Selon des modes de réalisation particuliers, l'éluant séparé à partir du premier flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du deuxième flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du troisième flux enrichi en premier PUFA est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en deuxième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en troisième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99% .

Selon des modes de réalisation particuliers, l'éluant séparé à partir du flux enrichi en quatrième acide gras est recyclé à plus de 50%, de préférence à plus de 60%, de préférence à plus de 70%, de préférence à plus de 80%, de préférence à plus de 90%, de préférence à plus de 95%, de préférence à plus de 98%, de préférence à plus de 99%.

De préférence, le produit d'alimentation qui est fourni en entrée de chaque unité de séparation chromatographique et qui est destiné à être séparé est aussi dépourvu de solvants que possible.

Ainsi :
- le mélange initial comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le premier flux enrichi en premier PUFA qui alimente la deuxième unité de séparation chromatographique 6 comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques ; et/ou
- le deuxième flux enrichi en premier PUFA qui alimente la troisième unité de séparation chromatographique 10 comprend moins de 80 % de solvants organiques, de préférence moins de 60 % ou moins de 40 % ou moins de 20 % ou moins de 10 % ou moins de 5 % ou moins de 2 % ou moins de 1 % de solvants organiques, et de manière plus particulièrement préférée est un mélange d'acides gras essentiellement dépourvu de solvants organiques.

Le procédé selon l'invention prévoit optionnellement une étape d'élimination (ou de réduction de la quantité) des composés oxygénés après la séparation chromatographique, ou après les séparations chromatographiques.

De préférence, cette étape n'est pas une étape de séparation chromatographique, et n'est pas mise en œuvre dans une unité de séparation chromatographique.

De préférence, cette étape ne sépare essentiellement pas le premier PUFA d'autres acides gras présents dans le flux (à l'exception des composés oxygénés du type aldéhydes et peroxydes).

L'étape d'élimination des composés oxygénés peut être mise en œuvre dans une unité de traitement 14, alimentée directement ou indirectement par la troisième conduite de flux enrichi en premier PUFA 12 (ou par la deuxième conduite de flux enrichi en premier PUFA 9 s'il n'y a que deux étapes de séparation chromatographique, ou par la première conduite de flux enrichi en premier PUFA 5 s'il n'y a qu'une seule étape de séparation chromatographique). En sortie de cette unité est connectée une conduite de collecte de premier PUFA purifié 15.

L'unité de traitement 14 peut notamment être une unité de distillation moléculaire ou évaporateur à court trajet. Un évaporateur à court trajet est équipé d'un condenseur intérieur et peut produire des évaporations avec un temps de résidence de préférence inférieur à 1000 s, de préférence inférieur à 100 s, de préférence inférieur à 10 s, sous une pression préférence inférieure à 10 mbar, de préférence inférieure à 1 mbar, de préférence inférieure à 0,1 mbar, de préférence inférieure à 0,01 mbar, de préférence inférieure à 0,001 mbar, à une température inférieure ou égale à 200°C, de préférence inférieure ou égale à 150°C, de préférence inférieure ou égale à 120°C, de préférence inférieure ou égale à 100°C, de préférence inférieure ou égale à 80°C.

Alternativement l'unité de traitement 14 peut être une unité de mise en contact avec un substrat d'adsorption.

Le substrat d'adsorption est tout substrat susceptible d'adsorber des composés oxygénés tels que les peroxydes et les composés aldéhydiques. Il peut être choisi par exemple parmi la silice, l'alumine, le charbon actif et les dérivés de ceux-ci, notamment les gels de silice, les silicates, aluminates et aluminosilicates. Une argile telle que la bentonite est un exemple de substrat approprié, de même que la terre de diatomée.

L'adsorption peut être effectuée en discontinu, c'est-à-dire en *« batch* », ou en continu, par percolation à travers un lit d'adsorbant. De préférence, les acides gras ne sont pas dilués au cours de cette étape, et en particulier aucun solvant n'est ajouté. La mise en contact peut durer par exemple de 5 minutes à 24 heures, et notamment de 10 minutes à 10 heures, de 20 minutes à 5 heures, de 30 minutes à 2 heures, et de 45 minutes à 1h30.

La quantité d'adsorbant utilisée dépend de la nature de l'adsorbant et de sa capacité à capturer les composés oxygénés. Elle peut être par exemple de 1 à 1000 g d'adsorbant par kg de flux à traiter (mélange d'acides gras), notamment de 10 à 500 g/kg, et plus particulièrement de 25 à 200 g/kg.

A l'issue de la mise en contact, l'adsorbant est séparé du mélange d'acides gras, et ce dernier peut être filtré afin d'éviter toute contamination par de l'adsorbant résiduel.

De préférence, la liaison des composés oxygénés à l'adsorbant est essentiellement irréversible, c'est-à-dire que l'adsorbant n'est pas régénéré. Il est cependant possible de régénérer l'adsorbant, par traitement thermique par exemple, afin de limiter le volume et/ou le coût de traitement des déchets.

L'étape de traitement dans l'unité de traitement 14 peut permettre de réduire l'indice de peroxyde du flux traité d'au moins 25 %, de préférence d'au moins 50 %, de préférence d'au moins 75 %, de préférence d'au moins 80 % ou d'au moins 90 % ou d'au moins 95 % ou d'au moins 98 %.

L'étape de traitement dans l'unité de traitement 14 peut permettre de réduire l'indice d'anisidine du flux traité d'au moins 25 %, de préférence d'au moins 50 %, de préférence d'au moins 75 %, de préférence d'au moins 80 % ou d'au moins 90 % ou d'au moins 95 % ou d'au moins 98 %.

L'indice de peroxyde mesure la quantité de composés peroxydes dans un mélange d'acides gras. La méthode d'analyse utilisée est de préférence Ph Eur 2.5.5 met A.

L'indice d'anisidine mesure la quantité de composés aldéhydiques dans un mélange d'acides gras. La méthode d'analyse utilisée est de préférence Ph Eur 2.5.36.

Selon un mode de réalisation, l'indice de peroxyde du flux issu de l'étape de traitement (produit récolté dans la conduite de collecte de premier PUFA purifié 15) est inférieur ou égal à 10, ou inférieur ou égal à 9, ou inférieur ou égal à 8, ou inférieur ou égal à 7, ou inférieur ou égal à 6, ou inférieur ou égal à 5, ou inférieur ou égal à 4, ou inférieur ou égal à 3, ou inférieur ou égal à 2, ou inférieur ou égal à 1,5, ou inférieur ou égal à 1.

Selon un mode de réalisation, l'indice d'anisidine du flux issu de l'étape de traitement (produit récolté dans la conduite de collecte de premier PUFA purifié 15) est inférieur ou égal à 20, ou inférieur ou égal à 18, ou inférieur ou égal à 16, ou inférieur ou égal à 14, ou inférieur ou égal à 12, ou inférieur ou égal à 10, ou inférieur ou égal à 9, ou inférieur ou égal à 8, ou inférieur ou égal à 7, ou inférieur ou égal à 6, ou inférieur ou égal à 5.

Une autre étape de traitement analogue à celle décrite ci-dessus, et plus particulièrement une étape de distillation moléculaire, peut également être prévue en amont, notamment avant toute étape de séparation chromatographique.

### Produit obtenu

Selon un mode de réalisation, le premier PUFA est un acide gras omega-3.

Selon des modes de réalisation différents, le premier PUFA peut être l'EPA, ou le DHA, ou l'ARA, ou le DPA, ou le SDA.

Selon un mode de réalisation, le premier PUFA est l'EPA, le deuxième acide gras est un acide gras saturé ou mono-insaturé, le troisième acide gras est le DHA ou le SDA, et le quatrième acide gras est celui du DHA et du SDA qui n'est pas le troisième acide gras. Par exemple, le troisième acide gras et le DHA et le quatrième acide gras est le SDA.

La concentration en premier PUFA dans le produit final obtenu à l'issue du procédé (récolté dans la conduite de collecte de premier PUFA purifié 15, ou dans la troisième conduite de collecte de flux enrichi en premier PUFA 12 s'il n'y a pas d'étape d'élimination des composés oxygénés, ou encore dans la deuxième conduite de collecte de flux enrichi en premier PUFA 9 s'il n'y a ni troisième étape de séparation chromatographique ni étape d'élimination des composés oxygénés, ou encore dans la première conduite de collecte de flux enrichi en premier PUFA 5 s'il n'y ni deuxième étape de séparation chromatographique, ni troisième étape de séparation chromatographique, ni étape d'élimination des composés oxygénés) peut être supérieure ou égale à environ 80 %, de préférence supérieure ou égale à environ 90%, ou à environ 95 %, ou à environ 97 %, ou à environ 98 %, ou à environ 99 % (par rapport au total des acides gras).

La concentration en deuxième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

La concentration en troisième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

La concentration en quatrième acide gras dans ce produit final peut être inférieure ou égale à environ 1 %, ou à environ 0,1%, ou à environ 0,05 %, ou à environ 0,03 %, ou à environ 0,01% (par rapport au total des acides gras).

Par exemple, le produit final obtenu à l'issue du procédé, peut contenir de l'EPA dans une proportion supérieure ou égale à environ 80 %, ou supérieure ou égale à environ 95 %, ou supérieure ou égale à environ 97 %, ou supérieure ou égale à environ 98 %, ou supérieure ou égale à environ 99% (par rapport au total des acides gras); ainsi que du DHA dans une proportion inférieure ou égale à environ 1 %, ou inférieure ou égale à environ 0,1%, ou inférieure ou égale à environ 0,05 %, ou inférieure ou égale à environ 0,03 %, ou inférieure ou égale à environ 0,01%.

L'huile enrichie en premier PUFA est stockée à l'abri de l'air et de la lumière avant son conditionnement et/ou son utilisation, comprenant par exemple la formulation finale et/ou l'encapsulation.

Selon un mode de réalisation, le produit final est combiné avec un véhicule pharmaceutiquement et/ou diététiquement acceptable et/ou des excipients et/ou diluants. Ce produit peut ainsi être formulé par exemple sous forme de gélules, capsules ou comprimés (ou sous toute autre forme adaptée à une administration orale ou topique ou parentérale).

Chaque forme de dosage individuelle (par exemple capsule ou gélule) peut contenir par exemple de 250 à 1500 mg, de préférence de 300 à 1000 mg du produit ci-dessus.

Le produit peut ainsi être utilisé pour la préparation d'une composition pharmaceutique pour la prévention et/ou le traitement et/ou la prophylaxie de facteurs de risques pour les maladies cardiovasculaires, comme l'hypertriglycéridémie, l'hypercholestérolémie et l'hypertension ; et de maladies cardiovasculaires comme l'arythmie, la fibrillation atriale et/ou ventriculaire, la décompensation et l'insuffisance cardiaque ; pour la prévention primaire et secondaire de l'infarctus et du ré-infarctus ; pour le traitement de toute autre pathologie pouvant être traitée par les PUFA susmentionnés, comme par exemple les maladies auto-immunes, la cholique ulcéreuse, les pathologies tumorales, les maladies du système nerveux, le vieillissement cellulaire, l'infarctus cérébral, les maladies ischémiques, le psoriasis.

Alternativement, le produit peut être utilisé dans des utilisations parapharmaceutiques, et notamment diététiques.

### EXEMPLES

Les exemples suivants illustrent l'invention sans la limiter.

### Exemple 1a - purification en une étape à l'échelle laboratoire (comparatif)

Dans cet exemple, un ester éthylique d'EPA à plus de 94% est obtenu à partir d'un mélange d'esters éthyliques contenant plus de 70% d'EPA.

L'EPA est purifié par une étape de chromatographie, sur silice phase inversée C18, avec des particules de 20 µm de diamètre en moyenne. Les conditions de chromatographie sont les suivantes :
- CYCLOJET™ équipé d'une colonne à compression dynamique axiale de 39 cm de long et de 5 cm de diamètre
- Eluant : méthanol/eau à 93/7 v/v.

On collecte une fraction contenant de l'EPA à environ 94 % (aire CPG), et moins de 0,1 % de DHA. Le taux de récupération de l'EPA dans cette fraction est supérieur à 95%. La productivité spécifique est de 2,76 kg d'EPA pur / kg phase stationnaire / jour. La consommation spécifique d'éluant est de 224 L/kg d'EPA pur.

Après concentration dans un évaporateur rotatif opéré sous vide, la fraction concentrée contient moins de 1% de solvants résiduels.

### Exemple 1b (comparatif)

Dans cet exemple, le mélange d'esters éthyliques contenant plus de 70% d'EPA de l'exemple précédent est purifié dans un système VARICOL™ équipé de 5 colonnes de 9 cm de long et 4,8 cm de diamètre, avec un éluant méthanol/eau à 93/7 v/v.

Le raffinat contient de l'EPA à environ 92% (aire CPG), et moins de 0,1 % de DHA. Le taux de récupération de l'EPA dans le raffinat est supérieur à 95 %. La productivité spécifique est de 2,46 kg d'EPA pur / kg de phase stationnaire / jour. La consommation spécifique d'éluant est de 258 L/kg d'EPA pur.

La productivité spécifique du CYCLOJET™ est donc de 12% supérieure à celle du VARICOL™, et sa consommation spécifique d'éluant est inférieure de 13% par rapport à celle du VARICOL™. De façon surprenante, le CYCLOJET™ conduit à des performances supérieures à celles du VARICOL™ pour cette purification en une étape.

### Exemple 2a : ultra-purification à l'échelle laboratoire par CYCLOJET™

Dans cet exemple, un ester éthylique d'EPA à plus de 96 % est obtenu par une étape de chromatographie à partir du produit obtenu à l'exemple 1a.

L'EPA est purifié sur silice phase inversée C18, avec des particules de 20 µm de diamètre en moyenne. Les conditions de chromatographie sont les suivantes :
- Cyclojet équipé d'une colonne à compression dynamique axiale de 39 cm de long et de 5 cm de diamètre.
- Eluant : acétone/eau à 79/21 v/v.

L'une des fractions collectées contient de l'EPA à environ 96,8% (aire CPG), et moins de 0,1% de DHA. Le taux de récupération de l'EPA dans l'extrait est d'environ 95 %. La productivité spécifique est de 3,58 kg d'EPA pur / kg de phase stationnaire / jour. La consommation spécifique d'éluant est de 153 L/kg d'EPA pur.

Après concentration dans un évaporateur rotatif opéré sous vide, la fraction concentrée contient moins de 1 % de solvants résiduels.

### Exemple 2b (comparatif)

Dans cet exemple, le raffinat concentré obtenu à l'exemple 1a est purifié dans un VARICOL™ équipé de 5 colonnes de 9 cm de long et 4,8 cm de diamètre, avec un éluant acétone/eau à 79/21 v/v.

L'extrait contient de l'EPA à environ 97 % (aire CPG), et moins de 0,1 % de DHA. Le taux de récupération de l'EPA dans l'extrait est d'environ 95 %. La productivité spécifique est de 3,35 kg d'EPA pur / kg de phase stationnaire / jour.

La productivité spécifique du Cyclojet est 7 % supérieure à celle du VARICOL™. De façon surprenante, le Cyclojet conduit à une meilleure productivité que le VARICOL™ pour la deuxième étape de purification.

### Exemple 3 - purification en trois étapes à l'échelle pilote

Dans cet exemple, un ester éthylique d'EPA à plus de 96 % est obtenu par trois étapes de chromatographie à partir d'un mélange d'esters éthyliques contenant plus de 50 % d'EPA.

L'EPA est purifié par trois étapes de chromatographie, sur silice phase inversée C18. Les conditions de chromatographie sont les suivantes :

### Etape 1 :

- VARICOL™ équipé de 5 colonnes de 9 cm de long et 59 cm de diamètre. Configuration des colonnes: 1/1,6/1,6/0,8 dans les zones 1/2/3/4 respectivement.
- Eluant : acétone/eau à 90/10 v/v.

Le raffinat contient de l'EPA à environ 70 % (aire CPG). Après concentration dans un évaporateur rotatif opéré sous vide, le raffinat concentré contient moins de 1 % de solvants résiduels.

### Etape 2 :

- Cyclojet équipé d'une colonne à compression dynamique axiale de 49 cm de long et de 30 cm de diamètre.
- Eluant : méthanol/eau à 93/7 v/v.

On collecte une fraction contenant de l'EPA à environ 94 % (aire CPG), et moins de 0,1 % de DHA. Le taux de récupération de l'EPA dans la fraction est supérieur à 95 %.

Après concentration dans un évaporateur rotatif opéré sous vide, la fraction concentrée contient moins de 1% de solvants résiduels.

### Etape 3 :

- Cyclojet équipé d'une colonne à compression dynamique axiale de 49 cm de long et de 30 cm de diamètre.
- Eluant : acétone/eau à 79/21 v/v.

On collecte une fraction contenant de l'EPA à environ 96.,8% (aire CPG), et moins de 0,1% de DHA. Le taux de récupération de l'EPA dans la fraction est supérieur à 95 %

Après concentration dans un évaporateur rotatif opéré sous vide, la fraction concentrée contient moins de 1% de solvants résiduels.

## Revendications

1. Procédé de purification d'un premier acide gras à partir d'un mélange initial comprenant en outre au moins un deuxième acide gras, un troisième acide gras et optionnellement un quatrième acide gras, le procédé comprenant :
- une première étape de séparation chromatographique en phase liquide, à partir du mélange initial, effectuée dans une première unité de séparation chromatographique, permettant de récupérer d'une part un premier flux enrichi en premier acide gras et d'autre part un flux enrichi en deuxième acide gras,
- une deuxième étape de séparation chromatographique en phase liquide, à partir du premier flux enrichi en premier acide gras, effectuée dans une deuxième unité de séparation chromatographique, permettant de récupérer d'une part un deuxième flux enrichi en premier acide gras et d'autre part un flux enrichi en troisième acide gras ;
- optionnellement, une troisième étape de séparation chromatographique en phase liquide, à partir du deuxième flux enrichi en premier acide gras, effectuée dans une troisième unité de séparation chromatographique, permettant de récupérer d'une part un troisième flux enrichi en premier acide gras et d'autre part un flux enrichi en quatrième acide gras ;
au moins l'une parmi la deuxième unité de séparation chromatographique et la troisième unité de séparation chromatographique étant une unité de séparation chromatographique à lit statique à colonne unique avec recyclage à l'état stationnaire,
dans lequel le premier acide gras est un premier acide gras polyinsaturé.

2. Procédé selon la revendication 1, dans lequel la première unité de séparation chromatographique est une unité de séparation chromatographique à lit statique à colonne unique avec recyclage à l'état stationnaire ; et/ou la deuxième unité de séparation chromatographique est une unité de séparation chromatographique à lit statique à colonne unique avec recyclage à l'état stationnaire ; et/ou la troisième unité de séparation chromatographique est une unité de séparation chromatographique à lit statique à colonne unique avec recyclage à l'état stationnaire.

3. Procédé selon la revendication 1 ou 2, dans lequel au moins deux unités parmi la première unité de séparation chromatographique, la deuxième unité de séparation chromatographique et la troisième unité de séparation chromatographique sont des unités de séparation chromatographique à lit statique à colonne unique avec recyclage à l'état stationnaire.

4. Procédé selon l'une des revendications 1 à 3, dans lequel au moins l'une parmi la première unité de séparation chromatographique, la deuxième unité de séparation chromatographique et la troisième unité de séparation chromatographique, est une unité de séparation chromatographique à plusieurs colonnes.

5. Procédé selon l'une des revendications 1 à 4, dans lequel :
- la première étape de séparation chromatographique est mise en œuvre avec un premier éluant qui est un éluant hydro-organique ; et/ou
- la deuxième étape de séparation chromatographique est mise en œuvre avec un deuxième éluant qui est un éluant hydro-organique ; et/ou
- le cas échéant, la troisième étape de séparation chromatographique est mise en œuvre avec un troisième éluant qui est un éluant hydro-organique.

6. Procédé selon la revendication 5, dans lequel :
- le premier éluant est un mélange cétone / eau;
- le deuxième éluant est un mélange alcool / eau;
- le cas échéant, le troisième éluant est un mélange cétone / eau .

7. Procédé selon l'une des revendications 1 à 6, dans lequel :
- la concentration massique du premier éluant en solvant(s) organique(s) est contrôlée à 2 % près; et/ou
- la concentration massique du deuxième éluant en solvant(s) organique(s) est contrôlée à 2 % près; et/ou
- le cas échéant, la concentration massique du troisième éluant en solvant(s) organique(s) est contrôlée à 2 % près.

8. Procédé selon l'une des revendications 1 à 7, dans lequel :
- le premier acide gras polyinsaturé est l'acide eicosapentaénoïque ; ou
- le premier acide gras polyinsaturé est l'acide docosahexaénoïque ; ou
- le premier acide gras polyinsaturé est l'acide arachidonique ; ou
- le premier acide gras polyinsaturé est l'acide docosapentaénoïque.

9. Procédé selon la revendication 8, dans lequel :
- le premier acide gras polyinsaturé est l'acide eicosapentaénoïque, et est récupéré à l'issue du procédé avec une pureté supérieure ou égale à 80 % ; ou
- le premier acide gras polyinsaturé est l'acide docosahexaénoïque, et est récupéré à l'issue du procédé avec une pureté supérieure ou égale à 80 % ; ou
- le premier acide gras polyinsaturé est l'acide arachidonique, et est récupéré à l'issue du procédé avec une pureté supérieure ou égale à 80 %, ou
- le premier acide gras polyinsaturé est l'acide docosapentaénoïque, et est récupéré à l'issue du procédé avec une pureté supérieure ou égale à 80 %.

10. Procédé selon l'une des revendications 1 à 9, comprenant ;
- au moins une étape de séparation chromatographique entre le premier acide gras et un ou des composés plus retenus que celui-ci; et/ou
- au moins une étape de séparation chromatographique entre le premier acide gras et un ou des composés moins retenus que celui-ci .

11. Procédé selon l'une des revendications 1 à 10, dans lequel au moins l'une parmi la première unité de séparation chromatographique, la deuxième unité de séparation chromatographique et la troisième de séparation chromatographique comprend une colonne de séparation présentant une longueur supérieure ou égale à 5 cm, ou à 10 cm, ou à 20 cm, ou à 25 cm, ou à 30 cm, ou à 40 cm, ou à 50 cm, ou à 60 cm ; et/ou présentant un diamètre supérieur ou égal à 10 cm, ou à 20 cm, ou à 25 cm, ou à 30 cm, ou à 40 cm, ou à 50 cm, ou à 60 cm.

12. Procédé selon l'une des revendications 1 à 11, dans lequel :
- le mélange initial qui alimente la première unité de séparation chromatographique comprend moins de 80 % de solvants organiques; et/ou
- le premier flux enrichi en premier acide gras qui alimente la deuxième unité de séparation chromatographique comprend moins de 80 % de solvants organiques; et/ou
- le cas échéant, le deuxième flux enrichi en premier acide gras qui alimente la troisième unité de séparation chromatographique comprend moins de 80 % de solvants organiques.

13. Installation de purification d'un premier acide gras à partir d'un mélange initial, l'installation comprenant :
- une première unité de séparation chromatographique en phase liquide (3), alimentée par une conduite d'amenée de mélange initial (1), et à laquelle sont connectées en sortie d'une part une première conduite de flux enrichi en premier acide gras (5) et d'autre part une conduite de flux enrichi en deuxième acide gras (4) ;
- une deuxième unité de séparation chromatographique en phase liquide (6), alimentée par la première conduite de flux enrichi en premier acide gras (5), et à laquelle sont connectées en sortie d'une part une deuxième conduite de flux enrichi en premier acide gras (9) et d'autre part une conduite de flux enrichi en troisième acide gras (8) ;
- optionnellement, une troisième unité de séparation chromatographique en phase liquide (10), alimentée par la deuxième conduite de flux enrichi en premier acide gras (9), et à laquelle sont connectées en sortie d'une part une troisième conduite de flux enrichi en premier acide gras (12) et d'autre part une conduite de flux enrichi en quatrième acide gras (13) ;
dans laquelle au moins l'une parmi la deuxième unité de séparation chromatographique (6) et la troisième unité de séparation chromatographique (10) est une unité de séparation chromatographique à lit statique à colonne unique avec recyclage à l'état stationnaire,
dans lequel le premier acide gras est un premier acide gras polyinsaturé.

14. Installation selon la revendication 13, dans laquelle la première unité de séparation chromatographique (3) est une unité de séparation chromatographique à lit statique à colonne unique avec recyclage à l'état stationnaire ; et/ou la deuxième unité de séparation chromatographique (6) est une unité de séparation chromatographique à lit statique à colonne unique avec recyclage à l'état stationnaire ; et/ou la troisième unité de séparation chromatographique (10) est une unité de séparation chromatographique à lit statique à colonne unique avec recyclage à l'état stationnaire.

15. Installation selon la revendication 13 ou 14, dans laquelle au moins l'une parmi la première unité de séparation chromatographique (3), la deuxième unité de séparation chromatographique (6) et la troisième unité de séparation chromatographique (10) est une unité de séparation chromatographique à plusieurs colonnes.

16. Installation selon l'une des revendications 13 à 15, dans laquelle au moins l'une parmi la première unité de séparation chromatographique (3), la deuxième unité de séparation chromatographique (6) et la troisième unité de séparation chromatographique (10) comprend une colonne de séparation présentant une longueur supérieure ou égale à 5 cm, ou à 10 cm, ou à 20 cm, ou à 25 cm, ou à 30 cm, ou à 40 cm, ou à 50 cm, ou à 60 cm ; et/ou présentant un diamètre supérieur ou égal à 10 cm, ou à 20 cm, ou à 25 cm, ou à 30 cm, ou à 40 cm, ou à 50 cm, ou à 60 cm.

## Patentansprüche

1. Verfahren zur Reinigung einer ersten Fettsäure ab einem Ausgangsgemisch, umfassend ferner mindestens eine zweite Fettsäure, eine dritte Fettsäure und optional eine vierte Fettsäure, wobei das Verfahren umfasst:
- einen ersten Schritt des chromatografischen Trennens in flüssiger Phase, ab dem Ausgangsgemisch, durchgeführt in einer ersten chromatografischen Trenneinheit, wobei erlaubt wird, zum einen einen mit erster Fettsäure angereicherten ersten Strom und zum anderen einen mit zweiter Fettsäure angereicherten Strom zu gewinnen,
- einen zweiten Schritt des chromatografischen Trennens in flüssiger Phase, ab dem ersten mit erster Fettsäure angereicherten Strom, durchgeführt in einer zweiten chromatografischen Trenneinheit, wobei erlaubt wird, zum einen einen mit erster Fettsäure angereicherten zweiten Strom und zum anderen einen mit dritter Fettsäure angereicherten Strom zu gewinnen;
- optional einen dritten Schritt des chromatografischen Trennens in flüssiger Phase, ab dem mit erster Fettsäure angereicherten Strom, durchgeführt in einer dritten chromatografischen Trenneinheit, wobei erlaubt wird, zum einen einem mit erster Fettsäure angereicherten dritten Strom und zum anderen einen mit vierter Fettsäure angereicherten Strom zu gewinnen;
wobei mindestens eine von der zweiten chromatografischen Trenneinheit und der dritten chromatografischen Trenneinheit eine chromatografische Einkolonnen-Trenneinheit mit statischem Bett mit Rückführung in stationärem Zustand ist,
wobei die erste Fettsäure eine erste mehrfach ungesättigte Fettsäure ist.

2. Verfahren nach Anspruch 1, wobei die erste chromatografische Trenneinheit eine chromatografische Einkolonnen-Trenneinheit mit statischem Bett mit Rückführung in stationärem Zustand ist; und/oder die zweite chromatografische Trenneinheit eine chromatografische Einkolonnen-Trenneinheit mit statischem Bett mit Rückführung in stationärem Zustand ist; und/oder die dritte chromatografische Trenneinheit eine chromatografische Einkolonnen-Trenneinheit mit statischem Bett mit Rückführung in stationärem Zustand ist.

3. Verfahren nach Anspruch 1 oder 2, wobei mindestens zwei Einheiten von der ersten chromatografischen Trenneinheit, der zweiten chromatografischen Trenneinheit und der dritten chromatografischen Trenneinheit chromatografische Einkolonnen-Trenneinheiten mit statischem Bett mit Rückführung in stationärem Zustand sind.

4. Verfahren nach einem der Ansprüche 1 bis 3, wobei mindestens eine von der ersten chromatografischen Trenneinheit, der zweiten chromatografischen Trenneinheit und der dritten chromatografischen Trenneinheit eine chromatografische Mehrkolonnen-Trenneinheit ist.

5. Verfahren nach einem der Ansprüche 1 bis 4, wobei:
- der erste Schritt des chromatografischen Trennens mit einem ersten Elutionsmittel durchgeführt wird, welches ein hydro-organisches Elutionsmittel ist; und/oder
- der zweite Schritt des chromatografischen Trennens mit einem zweiten Elutionsmittel durchgeführt wird, welches ein hydro-organisches Elutionsmittel ist; und/oder
- gegebenenfalls, der dritte Schritt des chromatografischen Trennens mit einem dritten Elutionsmittel durchgeführt wird, welches ein hydro-organisches Elutionsmittel ist.

6. Verfahren nach Anspruch 5, wobei:
- das erste Elutionsmittel ein Gemisch Keton / Wasser ist;
- das zweite Elutionsmittel ein Gemisch Alkohol / Wasser ist;
- gegebenenfalls, das dritte Elutionsmittel ein Gemisch Keton / Wasser ist.

7. Verfahren nach einem der Ansprüche 1 bis 6, wobei:
- die Massenkonzentration des ersten Elutionsmittels in organischem/n Lösungsmittel/n auf nahezu 2 % kontrolliert ist, und/oder;
- die Massenkonzentration des zweiten Elutionsmittels in organischem/n Lösungsmittel/n auf nahezu 2 % kontrolliert ist; und/oder
- gegebenenfalls, die Massenkonzentration des dritten Elutionsmittels in organischem/n Lösungsmittel/n auf nahezu 2 % kontrolliert ist.

8. Verfahren nach einem der Ansprüche 1 bis 7, wobei:
- die erste mehrfach ungesättigte Fettsäure die Eicosapentaensäure ist; oder
- die erste mehrfach ungesättigte Fettsäure die Docosahexaensäure ist; oder
- die erste mehrfach ungesättigte Fettsäure die Arachidonsäure ist; oder
- die erste mehrfach ungesättigte Fettsäure die Docosapentaensäure ist.

9. Verfahren nach Anspruch 8, wobei:
- die erste mehrfach ungesättigte Fettsäure die Eicosapentaensäure ist und am Ende des Verfahrens mit einer Reinheit von über oder gleich 80 % zurückgewonnen wird; oder
- die erste mehrfach ungesättigte Fettsäure die Docosahexaensäure ist und am Ende des Verfahrens mit einer Reinheit von über oder gleich 80 % zurückgewonnen wird; oder
- die erste mehrfach ungesättigte Fettsäure die Arachidonsäure ist und am Ende des Verfahrens mit einer Reinheit von über oder gleich 80 % zurückgewonnen wird; oder
- die erste mehrfach ungesättigte Fettsäure die Docosapentaensäure ist und am Ende des Verfahrens mit einer Reinheit von über oder gleich 80 % zurückgewonnen wird.

10. Verfahren nach einem der Ansprüche 1 bis 9, umfassend:
- mindestens einen Schritt des chromatografischen Trennens zwischen der ersten Fettsäure und einer oder Verbindungen, die mehr als diese zurückgehalten wurden, und/oder
- mindestens einen Schritt des chromatografischen Trennens zwischen der ersten Fettsäure und einer oder Verbindungen, die weniger als diese zurückgehalten wurden.

11. Verfahren nach einem der Ansprüche 1 bis 10, wobei mindestens eine von der ersten chromatografischen Trenneinheit, der zweiten chromatografischen Trenneinheit und der dritten chromatografischen Trenneinheit eine Trennkolonne umfasst, die eine Länge aufweist, die größer oder gleich 5 cm oder 10 cm oder 20 cm oder 25 cm oder 30 cm oder 40 cm oder 50 cm oder 60 cm beträgt; und/oder einen Durchmesser aufweist, der größer oder gleich 10 cm oder 20 cm oder 25 cm oder 30 cm oder 40 cm oder 50 cm oder 60 cm ist.

12. Verfahren nach einem der Ansprüche 1 bis 11, wobei:
- das Ausgangsgemisch, das die erste chromatografische Trenneinheit versorgt, unter 80 % organische Lösungsmittel umfasst, und/oder
- der erste mit erster Fettsäure angereicherte Strom, der die zweite chromatografische Trenneinheit versorgt, unter 80 % organische Lösungsmittel umfasst, und/oder
- gegebenenfalls, der zweite mit erster Fettsäure angereicherte Strom, der die dritte chromatografische Trenneinheit versorgt, unter 80 % organische Lösungsmittel umfasst.

13. Anlage zur Reinigung einer ersten Fettsäure ab einem Ausgangsgemisch, wobei die Anlage umfasst:
- eine erste chromatografische Trenneinheit in flüssiger Phase (3), die von einer Ausgangsgemisch-Zufuhrleitung (1) versorgt wird, und an die am Auslass zum einen eine erste Leitung von mit erster Fettsäure angereichertem Strom (5) und zum anderen eine Leitung von mit zweiter Fettsäure (4) angereichertem Strom angeschlossen sind;
- eine zweite chromatografische Trenneinheit in flüssiger Phase (6), die von einer ersten Leitung von mit erster Fettsäure angereichertem Strom (5) versorgt wird, und an die am Auslass zum einen eine zweite Leitung von mit erster Fettsäure angereichertem Strom (9) und zum anderen eine Leitung von mit dritter Fettsäure (8) angereichertem Strom angeschlossen sind;
- optional, eine dritte chromatografische Trenneinheit in flüssiger Phase (10), die von einer zweiten Leitung von mit erster Fettsäure angereichertem Strom (9) versorgt wird, und an die am Auslass zum einen eine dritte Leitung von mit erster Fettsäure angereichertem Strom (12) und zum anderen eine Leitung von mit vierter Fettsäure (13) angereichertem Strom angeschlossen sind;
wobei mindestens eine von der zweiten chromatografischen Trenneinheit (6) und der dritten chromatografischen Trenneinheit (10) eine chromatografische Einkolonnen-Trenneinheit mit statischem Bett mit Rückführung in stationärem Zustand ist,
wobei die erste Fettsäure eine erste mehrfach ungesättigte Fettsäure ist.

14. Anlage nach Anspruch 12, wobei die erste chromatografische Trenneinheit (3) eine chromatografische Einkolonnen-Trenneinheit mit statischem Bett mit Rückführung in stationärem Zustand ist; und/oder die zweite chromatografische Trenneinheit (6) eine chromatografische Einkolonnen-Trenneinheit mit statischem Bett mit Rückführung in stationärem Zustand ist; und/oder die dritte chromatografische Trenneinheit (10) eine chromatografische Einkolonnen-Trenneinheit mit statischem Bett mit Rückführung in stationärem Zustand ist.

15. Anlage nach Anspruch 13 oder 14, wobei mindestens eine von der ersten chromatografischen Trenneinheit (3), der zweiten chromatografischen Trenneinheit (6) und der dritten chromatografischen Trenneinheit (10) eine chromatografische Mehrkolonnen-Trenneinheit ist.

16. Anlage nach einem der Ansprüche 13 bis 15, wobei mindestens eine von der ersten chromatografischen Trenneinheit (3), der zweiten chromatografischen Trenneinheit (6) und der dritten chromatografischen Trenneinheit (10) eine Trennkolonne umfasst, die eine Länge aufweist, die größer oder gleich 5 cm oder 10 cm oder 20 cm oder 25 cm oder 30 cm oder 40 cm oder 50 cm oder 60 cm beträgt; und/oder einen Durchmesser aufweist, der größer oder gleich 10 cm oder 20 cm oder 25 cm oder 30 cm oder 40 cm oder 50 cm oder 60 cm ist.

## Claims

1. Method for purifying a first fatty acid using an initial mixture further comprising at least one second fatty acid, a third fatty acid and optionally a fourth fatty acid, with the method comprising:
- a first step of chromatographic separation in liquid phase, using the initial mixture, carried out in a first unit for chromatographic separation, making it possible to recover on the one hand a first flow enriched with a first fatty acid and on the other hand a flow enriched with a second fatty acid,
- a second step of chromatographic separation in liquid phase, using the first flow enriched with a first fatty acid, carried out in a second chromatographic separation unit, making it possible to recover on the one hand a second flow enriched with a first fatty acid and on the other hand a flow enriched with a third fatty acid;
- optionally, a third step of chromatographic separation in liquid phase, using the second flow enriched with a first fatty acid, carried out in a third chromatographic separation unit, making it possible to recover on the one hand a third flow enriched with a first fatty acid and on the other hand a flow enriched with a fourth fatty acid;
at least one among the second unit for chromatographic separation and the third unit for chromatographic separation being a static bed chromatographic separation unit with a single column with recycling in stationary state,
wherein the first fatty acid is a first polyunsaturated fatty acid.

2. Method according to claim 1, wherein the first unit for chromatographic separation is a static bed chromatographic separation unit with a single column with recycling in stationary state; and/or the second unit for chromatographic separation is a static bed chromatographic separation unit with a single column with recycling in stationary state; and/or the third unit for chromatographic separation is a static bed chromatographic separation unit with a single column with recycling in stationary state.

3. Method according to claim 1 or 2, wherein at least two units among the first unit for chromatographic separation, the second unit for chromatographic separation and the third unit for chromatographic separation are static bed chromatographic separation units with a single column with recycling in stationary state.

4. Method according to one of claims 1 to 3, wherein at least one among the first unit for chromatographic separation, the second unit for chromatographic separation and the third unit for chromatographic separation, is a chromatographic separation unit with several columns.

5. Method according to one of claims 1 to 4, wherein:
- the first step of chromatographic separation is implemented with a first eluent which is a hydro-organic eluent; and/or
- the second step of chromatographic separation is implemented with a second eluent which is a hydro-organic eluent; and/or
- where applicable, the third step of chromatographic separation unit is implemented with a third eluent which is a hydro-organic eluent.

6. Method according to claim 5, wherein:
- the first eluent is a ketone / water mixture;
- where applicable, the second eluent is an alcohol / water mixture;
- where applicable, preferably, the third eluent is a ketone / water mixture.

7. Method according to one of claims 1 to 6, wherein:
- the mass concentration of the first eluent in organic solvent(s) is controlled to the nearest 2%; and/or
- the mass concentration of the second eluent in organic solvent(s) is controlled to the nearest 2%; and/or
- where applicable, the mass concentration of the third eluent in organic solvent(s) is controlled to the nearest 2%.

8. Method according to one of claims 1 to 7, wherein :
- the first polyunsaturated fatty acid is eicosapentaenoic acid; or
- the first polyunsaturated fatty acid is docosahexaenoic acid; or
- the first polyunsaturated fatty acid is arachidonic acid; or
- the first polyunsaturated fatty acid is docosapentaenoic acid.

9. Method according to claim 8, wherein :
- the first polyunsaturated fatty acid is eicosapentaenoic acid, and is recovered at the end of the method with a purity greater than or equal to 80%; or
- the first polyunsaturated fatty acid is docosahexaenoic acid, and is recovered at the end of the method with a purity greater than or equal to 80%; or
- the first polyunsaturated fatty acid is arachidonic acid, and is recovered at the end of the method with a purity greater than or equal to 80%; or
- the first polyunsaturated fatty acid is docosapentaenoic acid, and is recovered at the end of the method with a purity greater than or equal to 80%.

10. Method according to one of claims 1 to 9, comprising:
- at least one step of chromatographic separation between the first fatty acid and a compound or compounds that are more retained than the latter; and/or
- at least one step of chromatographic separation between the first fatty acid and a compound or compounds that are less retained than the latter.

11. Method according to one of claims 1 to 10, wherein at least one among the first unit for chromatographic separation, the second unit for chromatographic separation and the third chromatographic separation comprises a separation column that has a length greater than or equal to 5 cm, or to 10 cm, or to 20 cm, or to 25 cm, or to 30 cm, or to 40 cm, or to 50 cm, or to 60 cm; and/or that has a diameter greater than or equal to 10 cm, or to 20 cm, or to 25 cm, or to 30 cm, or to 40 cm, or to 50 cm, or to 60 cm.

12. Method according to one of claims 1 to 11, wherein:
- the initial mixture which supplies the first unit for chromatographic separation comprises less than 80% of organic solvents; and/or
- the first flow enriched with a first fatty acid which supplies the second unit for chromatographic separation comprises less than 80% of organic solvents; and/or
- where applicable, the second flow enriched with a first fatty acid which supplies the third unit for chromatographic separation comprises less than 80% of organic solvents.

13. Installation for purifying a first fatty acid using an initial mixture, with the installation comprising:
- a first unit for chromatographic separation in liquid phase (3), supplied by a supply duct of initial mixture (1), and to which are connected at the outlet on the one hand a first duct of flow enriched with a first fatty acid (5) and on the other hand a duct of flow enriched with a second fatty acid (4);
- a second chromatographic separation unit in liquid phase (6), supplied by the first duct of flow enriched with a first fatty acid (5), and to which are connected at the outlet on the one hand a second duct of flow enriched with a first fatty acid (9) and on the other hand a duct of flow enriched with a third fatty acid (8);
- optionally, a third chromatographic separation unit in liquid phase (10), supplied by the second duct of flow enriched with a first fatty acid (9), and to which are connected at the outlet on the one hand a third duct of flow enriched with a first fatty acid (12) and on the other hand a duct of flow enriched with a fourth fatty acid (13);
wherein at least one among the second unit for chromatographic separation (6) and the third unit for chromatographic separation (10) is a static bed chromatographic separation unit with a single column with recycling in stationary state,
wherein the first fatty acid is a first polyunsaturated fatty acid.

14. Installation according to claim 13, wherein the first unit for chromatographic separation (3) is a static bed chromatographic separation unit with a single column with recycling in stationary state; and/or the second unit for chromatographic separation (6) is a static bed chromatographic separation unit with a single column with recycling in stationary state; and/or the third unit for chromatographic separation (10) is a static bed chromatographic separation unit with a single column with recycling in stationary state.

15. Installation according to claim 13 or 14, wherein at least one among the first unit for chromatographic separation (3), the second unit for chromatographic separation (6) and the third unit for chromatographic separation (10), is a chromatographic separation unit with several columns.

16. Installation according to one of claims 13 to 15, wherein at least one among the first unit for chromatographic separation (3), the second unit for chromatographic separation (6) and the third chromatographic separation unit (10) comprises a separation column that has a length greater than or equal to 5 cm, or to 10 cm, or to 20 cm, or to 25 cm, or to 30 cm, or to 40 cm, or to 50 cm, or to 60 cm; and/or that has a diameter greater than or equal to 10 cm, or to 20 cm, or to 25 cm, or to 30 cm, or to 40 cm, or to 50 cm, or to 60 cm.
